# FASCICULE DE BREVET EUROPEEN

(11) **EP 0 652 194 B1**
(45) Date de publication et mention de la délivrance du brevet: **08.01.1997**
(21) Numéro de dépôt: 94402460.3
(22) Date de dépôt: 31.10.1994
(51) Int. Cl.: C07C 7/00, C07C 41/42

(54) **Procédé de séparation des composés oxygénés d'hydrocarbures, combinant une distillation et une perméation et son utilisation en éthérification**
Verfahren zur Trennung von Sauerstoffverbindungen von Kohlenwasserstoffen das Distillierung und Permeation sowie ihre Verwendung bei der Verätherung
Process for the separation of oxygenated compounds from hydrocarbons, combining a distillation step and a permeation step and its use in etherification

(30) Priorité: 05.11.1993 FR 9313319
(43) Date de publication de la demande: 10.05.1995
(73) Titulaire: INSTITUT FRANCAIS DU PETROLE, 92502 Rueil-Malmaison (FR)
(72) Inventeur: Streicher, Christian, F-92500 Rueil Malmaison (FR); Asselineau, Lionel, F-75017 Paris (FR)

(56) Documents cités:
- EP-A- 0 459 627
- US-A- 4 759 850

## Description

L'invention concerne un procédé de séparation de composés oxygénés (méthanol, diméthyléther, eau) d'un mélange d'hydrocarbures pouvant avoir de 3 à 8 atomes de carbone et son utilisation.

On sait que les alkyl tertioalkyléthers sont utilisés comme additifs à haut indice d'octane pour les essences sans plomb ou à teneur en plomb réduite.
Les alkyltertioalkyléthers les plus couramment utilisés sont obtenus par addition de méthanol sur une oléfine tertiaire.
Ainsi, par exemple le méthyltertiobutyléther (MTBE) est obtenu par addition d'une molécule de méthanol sur une molécule d'isobutène.
De même, le tertioamylméthyléther (TAME) est obtenu par addition d'une molécule de méthanol sur une molécule d'isoamylène.

Le procédé de synthèse de ces éthers consiste généralement à introduire dans un ou des réacteur(s) contenant un catalyseur approprié, en phase liquide une certaine quantité de méthanol ainsi que la ou les oléfine(s) tertiaire(s) qu'on cherche à faire réagir. Pour obtenir une conversion élevée des oléfines tertiaires on est amené à introduire un excès d'alcool qui doit ensuite être séparé de l'effluent de la section réactionnelle pour pouvoir être recyclé. Quant aux oléfines tertiaires elles sont généralement présentes dans un mélange d'hydrocarbures. Par exemple l'isobutène utilisé pour la synthèse du MTBE se trouve le plus souvent dans une coupe C4 de vapocraquage ou de craquage catalytique. Les hydrocarbures ainsi introduits dans la section réactionnelle avec la ou les oléfine(s) tertiaire(s) ne réagissent généralement pas ou peu et se retrouvent donc également dans l'effluent de la section réactionnelle.

L'effluent de la section réactionnelle est donc un mélange contenant l'éther produit de la réaction, l'excès d'alcool n'ayant pas réagi, les hydrocarbures n'ayant pas réagi ainsi que de faibles quantités de produits obtenus par des réactions secondaires comme par exemple l'éthérification du méthanol qui à partir de deux molécules de méthanol produit une molécule d'eau et une molécule de diméthyléther.

Divers procédés de séparation de ce mélange ont été proposés. Le procédé le plus couramment employé consiste à admettre l'effluent de la section réactionnelle dans une colonne à distiller produisant en fond un effluent contenant la totalité de l'éther produit, ainsi éventuellement qu'une partie de l'alcool présent dans l'effluent de la section réactionnelle. En tête de cette colonne à distiller, on obtient un raffinat constitué des hydrocarbures et d'une partie, voire préférentiellement de la totalité de l'alcool présent dans l'effluent de la section réactionnelle. Les teneurs en alcool généralement obtenues dans ce raffinat vont de 0,1 à 10 % poids, préférentiellement de 1 à 5 % poids. Cet alcool doit ensuite être séparé du raffinat pour pouvoir être recyclé à la section réactionnelle. En outre ce raffinat contient à l'état d'impuretés l'eau et le diméthyléther obtenus par réactions secondaires. Bien que présents en faibles quantités (de 0,01 à 0,1 % poids en général), ces produits oxygénés doivent souvent être eux aussi éliminés du raffinat. En effet une fois purifié, le raffinat sera le plus souvent utilisé comme charge pour d'autres réactions (alkylation par exemple) ; or les catalyseurs utilisés dans ces réactions ultérieures imposent en général pour ces charges des spécifications sévères de teneur en composés oxygénés totaux (de 1 à 50 ppm poids selon les cas).

Dans certains procédés, l'effluent de la section réactionnelle est envoyé dans une colonne de distillation catalytique, ce qui permet soit d'accroître le taux de conversion de la ou des oléfine(s) tertiaire(s), soit de réduire l'excès d'alcool utilisé. Quoiqu'il en soit, les raffinats obtenus en tête des colonnes de distillation catalytique ont des compositions analogues à celles des raffinats obtenus en tête des colonnes de distillation conventionnelles, avec simplement des teneurs en alcool légèrement inférieures.

L'élimination du méthanol contenu dans le raffinat n'est pas possible par simple distillation en raison de la formation d'azéotropes entre le méthanol et les hydrocarbures. Elle peut cependant être réalisée de différentes façons. Le procédé le plus couramment employé a été décrit dans le brevet US 3,726,942 et consiste à effectuer un lavage à l'eau de ce raffinat. Ce procédé bien qu'efficace conduit à effectuer ensuite une distillation du mélange eau/alcool ainsi obtenu pour pouvoir recycler l'alcool vers la section réactionnelle. Cette étape de distillation rend le procédé par lavage à l'eau assez coûteux tant du point de vue des investissements nécessaires que de celui de la consommation énergétique.

Un autre lavage à l'eau est illustré par le brevet EP-A-459 627.

D'autres variantes de ce procédé par lavage à l'eau ont été proposées. Par exemple le brevet US 4,118,425 décrit un procédé dans lequel c'est l'effluent de la section réactionnelle lui-même qui est lavé à l'eau avant d'être admis à la colonne de distillation, ce qui permet d'obtenir en tête de cette colonne un raffinat exempt d'alcool. Là aussi, la distillation du mélange eau/alcool produit lors de l'étape de lavage à l'eau est nécessaire pour le recyclage de l'alcool à la section réactionnelle.

De plus, le procédé par lavage à l'eau a pour inconvénient, quelles que soient les variantes utilisées, de ne pas éliminer du raffinat les impuretés oxygénées comme le diméthyléther. En outre, le raffinat lavé à l'eau se trouve bien entendu saturé en eau.

Le brevet US 4,740,631 décrit un procédé dans lequel le méthanol est éliminé du raffinat par adsorption sur un tamis moléculaire approprié (zéolithes) avec régénération séquentielle du tamis moléculaire par le mélange d'hydrocarbures constituant la charge de la section réactionnelle. Ce procédé offre l'avantage de supprimer l'étape de distillation requise par les procédés de lavage à l'eau. Malheureusement, comme tous les procédés d'adsorption, il s'agit d'un procédé discontinu, le tamis moléculaire utilisé devant être régénéré périodiquement. Cela implique l'utilisation d'au moins deux lits d'adsorption ainsi qu'une procédure opératoire relativement complexe pour effectuer les cycles alternés d'adsorption et de régénération. De plus, comme les procédés de lavage à l'eau, ce procédé d'adsorption ne permet pas d'éliminer le diméthyléther présent dans le raffinat. Là aussi des étapes ultérieures de purification, par exemple par adsorption sur d'autres tamis moléculaires ou par distillation, seront nécessaires pour atteindre les faibles teneurs en composés oxygénés totaux généralement requises.

Un procédé plus simple d'élimination du méthanol est proposé dans le brevet US 4,740,632. Dans ce procédé le raffinat est constitué d'un mélange de méthanol et d'hydrocarbures à 4 atomes de carbones. Ces hydrocarbures devant être envoyés dans une unité d'alkylation catalysée à l'acide sulfurique, on utilise l'acide sulfurique usagé produit par l'unité d'alkylation pour traiter le raffinat. On obtient ainsi une phase organique contenant les hydrocarbures sans méthanol qui sont envoyés à l'unité d'alkylation et une phase aqueuse contenant le méthanol ayant réagi avec l'acide sulfurique. Cette phase aqueuse est ensuite envoyée à une unité de régénération de l'acide permettant de récupérer ce dernier. Ce procédé bien que très simple s'avère cependant assez coûteux lorsque le raffinat à traiter contient plus de 0,5 % poids de méthanol, ce qui est généralement le cas, le méthanol ayant réagi avec l'acide ne pouvant pas être récupéré lors de la régénération de l'acide pour être recyclé à la section réactionnelle.

Une autre méthode pour séparer l'alcool présent dans le raffinat peut être de mettre le mélange à séparer au contact d'une membrane perméable sélectivement soit à l'alcool, ce qui est le cas le plus fréquent et aussi le plus avantageux compte-tenu des faibles quantités d'alcool à extraire, soit aux hydrocarbures.

Ainsi le brevet US 4,759,850 décrit une méthode permettant de séparer le méthanol d'un mélange d'hydrocarbures et/ou d'éthers par osmose inverse.

De meilleures sélectivités et par conséquent des produits plus purs ont cependant été obtenus en pervaporation. Dans ce procédé, le mélange à séparer est mis, en phase liquide à la température et à la pression appropriées, en contact avec l'une des faces d'une membrane dont l'autre face est maintenue sous vide. La membrane étant sélective pour l'un des constituants du mélange, celui-ci diffuse préférentiellement à travers la membrane. On récupère ainsi en aval de la membrane (côté sous vide) un perméat enrichi en ce constituant qui sera, selon les cas, comprimé ou condensé à basse température. En amont de la membrane, on obtient un rétentat qui est le mélange initial appauvri en ce constituant. Ce procédé du fait de sa sélectivité est particulièrement avantageux lorsqu'il s'agit de séparer des mélanges azéotropiques.

Les membranes de pervaporation utilisées actuellement sont pour l'essentiel des membranes sélectives pour l'eau dans des mélanges de produits organiques. Certaines membranes sélectivement perméables aux alcools dans des mélanges organiques ont cependant été décrites.

Ainsi les brevets US 4,798,674, 4,877,529, 4,960,519 et 5,152,898, le brevet allemand DE 4 234 521 et la demande de brevet européen EP-A-0 592 706 décrivent divers modes de réalisation de telles membranes et leur utilisation pour l'extraction par pervaporation, d'alcools ayant moins de trois atomes de carbone, préférentiellement le méthanol, de mélanges contenant d'autres composés organiques oxygénés tels des éthers, esters, aldéhydes, cétones.

Le brevet US 4,774,365 décrit un procédé de séparation de l'alcool présent dans l'effluent de la section réactionnelle d'un procédé d'éthérification mettant en oeuvre une membrane de pervaporation.

Dans une première variante du procédé, l'effluent de la section réactionnelle passe sur une membrane de pervaporation qui en extrait sélectivement l'alcool. L'alcool ainsi extrait est recyclé à la section réactionnelle. Le rétentat appauvri en alcool ainsi obtenu est ensuite distillé. Dans cette variante il est possible d'obtenir en fond de la colonne de distillation un éther exempt d'alcool. Cependant pour que le raffinat obtenu en tête de cette colonne de distillation soit entièrement exempt d'alcool, il est nécessaire que tout l'alcool présent dans l'effluent de la section réactionnelle ait été éliminé par l'unité de pervaporation. Or il est bien connu que la pervaporation, à l'instar de tous les procédés membranaires, devient très coûteuse lorsqu'il faut extraire les dernières traces d'un produit (en général descendre à des concentrations inférieures à 0,1 % n'est pas économique). Il est donc difficile par cette variante d'obtenir un raffinat à très faible teneur en alcool.

Ce même brevet propose une autre variante dans laquelle l'effluent de la section réactionnelle est directement envoyé sur la colonne à distiller. Une fraction liquide prélevée par un soutirage latéral sur cette colonne est envoyée sur une membrane de pervaporation qui extrait une partie de l'alcool présent dans cette fraction liquide. L'alcool extrait est recyclé vers la section réactionnelle tandis que le rétentat appauvri en alcool est renvoyé sur la colonne à distiller. Là aussi, cette variante permet bien d'obtenir en fond de colonne à distiller un éther exempt d'alcool. Cependant en raison de l'entraînement par azéotropie de l'alcool avec les hydrocarbures, il est difficile, même avec cette variante, d'obtenir en tête de la colonne à distiller un raffinat contenant moins de 0,1 % poids d'alcool résiduel (valeur donnée dans l'exemple 2 du brevet). Cela reste vrai pour toute combinaison des 2 variantes comme on peut le voir dans l'exemple n°2 du brevet.

De plus, sauf à disposer d'une membrane permettant l'extraction simultanée du méthanol et du diméthyléther d'un mélange contenant des hydrocarbures et/ou d'autres éthers, il apparaît clairement quelle que soit la variante utilisée, qu'un tel procédé ne permet pas d'éliminer ou de réduire les quantités de diméthyléther, présentes dans l'effluent de la section réactionnelle et qui se retrouvent dans le raffinat.

La présente invention a donc pour objet un procédé de séparation du méthanol d'un mélange d'hydrocarbures pouvant avoir de 3 à 8 atomes de carbone combinant une étape de distillation avec une étape de pervaporation de manière à obtenir la majeure partie (plus de 95 % préférentiellement plus de 99 %) de ces hydrocarbures à de très faibles, par exemple moins de 50 ppm poids, teneurs en méthanol résiduel.

Le procédé de la présente invention permet également, lorsque le mélange d'hydrocarbures et de méthanol à traiter contient du diméthyléther et/ou de l'eau d'éliminer ces produits de manière à obtenir ladite majeure partie des hydrocarbures à de très faibles teneurs en composés oxygénés totaux, par exemple moins de 50 ppm poids.

La présente invention a également pour objet un procédé de synthèse d'éthers à partir de méthanol et de mélanges d'hydrocarbures ayant de 3 à 8 atomes de carbone (coupes C4, C5, C6, C7 ou C8 et/ou leurs mélanges) dans lequel le procédé de séparation de l'invention est utilisé et dans lequel le méthanol produit par le procédé de séparation est recyclé vers la section réactionnelle.

Le procédé de la présente invention s'adresse plus particulièrement aux mélanges d'hydrocarbures (coupes C4 à C8 et/ou leurs mélanges) obtenus, dans les procédés d'éthérification utilisant le méthanol, après séparation de l'éther produit, par distillation et/ou distillation catalytique de l'effluent de la section réactionnelle. Ces mélanges contiennent généralement de 0,1 à 20 % poids de méthanol, préférentiellement de 0,5 à 5 % poids de méthanol. Ils contiennent également en général de 10 ppm poids à 1 % poids d'eau, préférentiellement de 100 à 1000 ppm poids d'eau, ainsi que de 10 ppm poids à 1 % poids de diméthyléther, préférentiellement de 100 à 1000 ppm poids de diméthyléther.

Plus précisément l'invention concerne un procédé de séparation de composés oxygénés, comprenant en majeure partie du méthanol et éventuellement une mineure partie d'eau et/ou de diméthyléther, d'un mélange d'hydrocarbures pouvant avoir de 3 à 8 atomes de carbone, contenant lesdits composés oxygénés, comprenant une étape de distillation du mélange dans des conditions de pression et de température appropriées dans une zone de distillation.

On recueille en tête de la zone de distillation un distillat gazeux comprenant du méthanol, le cas échéant de l'eau et/ou du diméthyléther, ainsi que des hydrocarbures. Ce distillat est condensé et recyclé au moins en partie.

On soutire latéralement de la zone de distillation une phase qui peut être gazeuse, liquide ou mixte et on l'envoie dans des conditions adéquates dans une zone de perméation comprenant au moins une membrane sélectivement perméable au méthanol; on réalise une étape de perméation de ladite phase.

On recueille en aval de la zone de perméation, au moins un perméat, de préférence en phase vapeur, enrichi en méthanol et en amont de la zone de perméation, au moins un rétentat, et on soutire en fond de la zone de distillation le mélange d'hydrocarbures purifiés.

Par zone de perméation, on entend une zone de pervaporation lorsqu'une phase liquide est mise en contact avec la face amont de la membrane ou une zone de perméation de vapeur lorsqu'il s'agit d'une phase gazeuse ou mixte.

Selon une caractéristique du procédé, on peut soutirer latéralement la phase d'au moins un plateau de la zone de distillation, dans lequel la concentration en méthanol est au moins égale à celle du méthanol dans le mélange d'hydrocarbures alimentant la zone de distillation et de préférence dans lequel la concentration en méthanol est sensiblement maximale.

La phase organique soutirée contient du méthanol et des hydrocarbures mais elle peut contenir aussi une mineure quantité d'eau et de diméthyléther. Dans ces conditions, si la membrane choisie est imperméable au diméthyléther, celui-ci est essentiellement recueilli dans le rétentat et recyclé. Par contre si la membrane choisie est perméable au diméthyléther, celui -ci peut se retrouver au moins en partie dans le perméat.

Selon une autre caractéristique du procédé, la pression dans la zone de perméation (exercée sur la face amont de la membrane) peut être supérieure, égale ou inférieure à la pression dans la zone de distillation.

Lorsqu'une phase liquide est soutirée latéralement, ladite phase liquide peut être portée aux conditions de températures et de pressions requises pour son admission dans la zone de pervaporation au moyen d'équipements appropriés: échangeurs de chaleur, pompes, vannes de détente.

Par contre, lorsqu'une phase gazeuse ou mixte est soutirée, elle sera préférentiellement admise directement dans la zone de perméation de vapeur. Elle peut cependant également être portée à des conditions de température et de pressions différentes et préférables pour son admission dans la zone de perméation de vapeur au moyen d'équipements appropriés, échangeurs de chaleur, pompes, compresseurs, vannes de détente.

Selon une autre caractéristique du procédé, le rétentat n'ayant pas traversé la membrane peut être recyclé dans la zone de distillation. Ce rétentat peut être obtenu après la zone de perméation en phase liquide, vapeur ou mixte. Il doit pour être recyclé dans la zone de distillation se trouver à une pression au moins supérieure à celle de la zone de distillation. Si ce n'est pas le cas, il peut y être porté au moyen d'équipements appropriés, pompes, compresseurs. Dans certains cas il peut être avantageux de modifier la température de ce rétentat avant son admission dans la zone de distillation en l'augmentant ou la diminuant par passage dans un ou des échangeur(s) de chaleur avant ou après son éventuel passage dans lesdits équipements de montée en pression.

Ce rétentat peut avantageusement être recyclé dans la zone de distillation à un plateau inférieur à celui du soutirage latéral de la phase prélevée et de préférence ce rétentat peut être recyclé au plateau dont la composition est sensiblement la même que celle du rétentat.

Selon une autre caractéristique du procédé, on peut condenser au moins en partie le distillat gazeux et recueillir au moins une partie d'une phase organique liquide qui sert de reflux, éventuellement une phase aqueuse et éventuellement une phase gazeuse ou liquide contenant du méthanol et du diméthyléther.

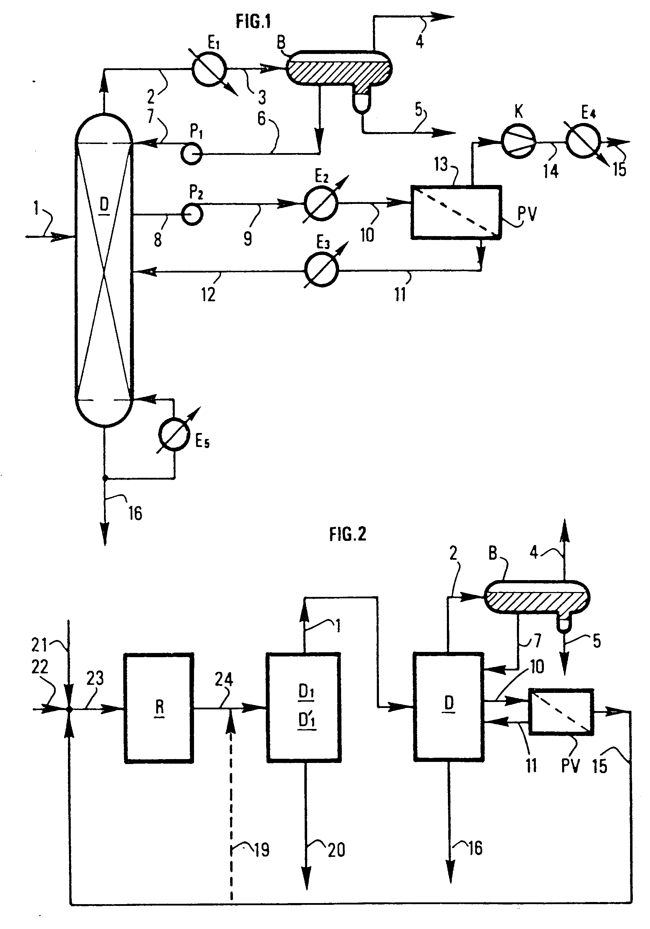

On décrit un mode avantageux de réalisation de l'invention en relation avec la figure 1.

Le mélange à séparer contenant par exemple jusqu'à 5 % poids de méthanol (cette quantité étant fonction de la coupe d'hydrocarbures introduite dans le réacteur d'éthérification) est envoyé par la ligne 1 dans la colonne à distiller D. Cette colonne opère généralement sous une pression supérieure à 1 bar, avantageusement entre 2 et 35 bar. Elle est chauffée par le rebouilleur E5. La température de fond est en général comprise entre 70 et 200°C. La température de tête est en général comprise entre 40 et 120°C. Le mélange à séparer est introduit dans la colonne D préférentiellement au point de bulle à la pression considérée, au plateau ayant la composition la plus voisine possible de celle dudit mélange, par exemple dans la partie supérieure de la colonne dans le cas de mélanges contenant de 1 à 5 % poids de méthanol.

En tête de colonne on récupère par la ligne 2 un distillat en phase vapeur constitué de 0,1 à 5 % poids d'eau lorsque le mélange à séparer en contient, de 0,5 à 10 % poids, c'est-à-dire la majorité du diméthyléther lorsque le mélange à séparer en contient, de 0,5 à 10 % poids de méthanol, le reste étant constitué d'hydrocarbures. Cette vapeur est ensuite condensée partiellement dans l'échangeur E1 puis amenée par la ligne 3 au ballon B. On obtient ainsi après décantation dans le ballon B, par la ligne 4 une vapeur résiduelle contenant de 0,5 à 10 % poids de méthanol, de 0,1 à 5 % poids d'eau lorsque le mélange à séparer en contient, de 0,5 à 20 % poids de diméthyléther lorsque le mélange à séparer en contient et des hydrocarbures. Les hydrocarbures perdus dans cette vapeur résiduelle représentent moins de 5 %, préférentiellement moins de 1 % des hydrocarbures présents dans le mélange à séparer. Cette vapeur résiduelle peut donc aisément être utilisée comme combustible sans que la perte en hydrocarbures ainsi consentie ne pénalise la rentabilité économique du procédé.

Lorsque le mélange à séparer contient de l'eau on récupère généralement la majeure partie de celle-ci au niveau du ballon B par la ligne 5, sous forme d'une phase liquide aqueuse contenant généralement de 1 à 70 % poids de méthanol et de 0,1 à 5 % poids d'hydrocarbures et/ou de diméthyléther, lorsque le mélange à séparer en contient.

La quantité de phase liquide aqueuse ainsi obtenue est fonction de la teneur en eau du mélange à séparer. Pour les mélanges habituellement rencontrés dans les procédés d'éthérification, qui contiennent moins de 0,1 % poids d'eau, cette phase liquide aqueuse représente généralement moins de 0,1 % poids de la quantité de mélange traité par le procédé. Elle peut donc aisément être envoyée soit vers une unité conventionnelle de traitement des eaux usées lorsqu'elle contient de faibles teneurs en méthanol, soit éliminée par combustion lorsqu'elle contient par exemple, plus de 50% poids de méthanol, là aussi sans que le coût du méthanol ainsi perdu et le coût du traitement de cette phase aqueuse ne pénalisent la rentabilité économique du procédé.

Enfin, la majeure partie de la phase liquide obtenue au niveau du ballon B est une phase liquide organique dont la composition est voisine de celle de la vapeur obtenue en tête de la colonne à distiller D. Cette phase liquide organique est amenée à la pompe P1 via la ligne 6 puis injectée comme reflux en tête de la colonne D, via la ligne 7. Les taux de reflux ainsi utilisés représentent généralement de 0,3 à 3 fois, préférentiellement de 0,5 à 1,5 fois le débit massique du mélange à séparer constituant la charge de la colonne à distiller D.

Une fraction liquide est prélevée par la ligne 8 sur un soutirage latéral de la colonne à distiller D. Ce soutirage latéral est préférentiellement placé au niveau du plateau où la concentration en méthanol dans la phase liquide est maximale. La fraction liquide ainsi prélevée a généralement une concentration en méthanol supérieure à celle du mélange à séparer qui constitue la charge de la colonne à distiller. Le débit massique de la fraction liquide ainsi prélevée peut être supérieur, égal ou inférieur à celui de la charge alimentant la colonne à distiller et ce en fonction des concentrations en méthanol dans ladite fraction liquide et dans le rétentat de l'unité de pervaporation PV.

Cette fraction liquide est ensuite amenée grâce à la pompe P2 à la pression requise pour être envoyée à l'unité de pervaporation PV, puis, recyclée vers la colonne à distiller D. Puis par la ligne 9 la fraction liquide est refoulée vers l'échangeur E2 où elle est portée à une température permettant son traitement par l'unité de pervaporation. Cette température dépend notamment de la membrane utilisée dans l'unité de pervaporation. Elle est généralement comprise entre 50 et 200°C et avantageusement entre 80 et 120°C.

La fraction liquide parvient ensuite par la ligne 10 à l'unité de pervaporation PV.

L'unité de pervaporation PV consiste en un ensemble d'un ou plusieurs modules, disposés en série et/ou en parallèle à l'intérieur desquels se trouve une membrane sélectivement perméable au méthanol comme par exemple l'une de celles décrites dans les brevets US 4,798,674; 4,877,529; 4,960,519 ou 5,152,898, dans le brevet allemand DE 4 234 521 ou la demande de brevet européen EP-A-0 592 706. Cette membrane sépare chaque module en un ou plusieurs compartiment(s) amont(s) à l'intérieur du (des) quel(s) circule la fraction liquide et en un ou plusieurs compartiment(s) aval(s) à l'intérieur du (des) quel(s) circulent en phase vapeur l'ensemble des produits ayant traversé ladite membrane, cette phase vapeur étant appelée perméat. Le ou les compartiment(s) aval(s) du ou des différent(s) module(s) se trouve(nt) à une (ou des) pression(s) inférieure(s) à celle du liquide circulant en amont de la membrane. Cette (ces) pression(s) est (sont) généralement inférieure(s) à 1 bar(abs.).(1 bar = 10⁵Pa).

Entre deux modules en série, l'unité de pervaporation comporte généralement des réchauffeurs intermédiaires (non représentés sur la figure 1) qui permettent de compenser le refroidissement du liquide circulant en amont de la membrane provoqué par l'évaporation d'une partie des constituants dudit liquide.

L'ensemble des perméats recueillis sur chacun des modules est, après compression éventuelle par le compresseur K, condensé dans l'échangeur E4. Bien que sur la figure 1 on n'ait représenté qu'un seul compresseur K et qu'un seul échangeur E4, il est clair que lorsque l'unité de pervaporation comporte plusieurs modules produisant des perméats en phase vapeur sous différentes pressions, plusieurs compresseurs et/ou échangeurs en parallèle et/ou en série peuvent être utilisés.

On récupère ainsi au niveau de la ligne 15 un perméat en phase liquide constitué généralement de plus de 50 % poids, préférentiellement plus de 90 % poids de méthanol. La composition exacte de ce perméat dépend de la sélectivité et de la surface de la membrane utilisée dans l'unité de pervaporation PV ainsi que de la concentration en méthanol du liquide alimentant cette unité par la ligne 10. Généralement plus de 95 %, préférentiellement plus de 99 % du méthanol présent dans la charge de la colonne à distiller D se trouve dans ce perméat liquide.

Le liquide ayant circulé en amont de la membrane, nommé rétentat, est recueilli appauvri en méthanol par la ligne 11. Ce liquide est porté dans l'échangeur E3 à une température permettant son retour sur la colonne à distiller D, généralement à la température du plateau où ledit liquide sera réinjecté. Ce liquide alimente ensuite la colonne à distiller D par la ligne 12, préférentiellement au plateau dont la composition est la plus proche dudit liquide.

Enfin, le résidu de distillation, sortant en fond de colonne par la ligne 16 est constituée essentiellement des hydrocarbures purifiés. Les conditions opératoires concernant les divers débits d'alimentation et l'efficacité de la colonne sont en général ajustées de manière que plus de 95% et préférentiellement plus de 99 % des hydrocarbures présents dans le mélange à séparer soient ainsi obtenus à des teneurs en eau inférieures à 10 ppm poids, préférentiellement inférieures à 1 ppm poids, à des teneurs en méthanol inférieures à 50 ppm poids, préférentiellement inférieures à 5 ppm poids et à des teneurs en diméthyléther inférieures à 100 ppm poids, préférentiellement inférieures à 10 ppm poids.

L'un des avantages du procédé ainsi décrit est sa très grande simplicité puisqu'il permet avec un nombre minimum d'unités (une colonne à distiller et une unité de perméation) de séparer à la fois le méthanol, l'eau et le diméthyléther d'un mélange d'hydrocarbures en obtenant lesdits hydrocarbures à des teneurs très faibles en ces composés oxygénés, rendant ainsi inutiles des étapes ultérieures de purification desdits hydrocarbures.

Un autre avantage du procédé vient du fait qu'il peut fonctionner avec une membrane sélectivement perméable au méthanol, sans que cette membrane soit nécessairement sélectivement perméable à l'eau et/ou au diméthyléther. Bien entendu le procédé de la présente invention pourrait fonctionner avantageusement avec une membrane qui serait sélectivement perméable au méthanol ainsi qu'à l'eau et/ou au diméthyléther. Si la quasi-totalité des membranes connues pour être sélectivement perméables au méthanol le sont aussi à l'eau, on n'en connaît cependant pas à ce jour qui le soient également au diméthyléther.

Enfin un autre avantage du procédé de la présente invention vient de ce que malgré les très faibles teneurs en méthanol obtenues dans le produit final, I'unité de perméation fonctionne dans une plage de concentrations en méthanol nettement plus élevées, généralement comprises entre 0,1 % poids, préférentiellement 0,5 % poids, pour le rétentat et 20 % poids pour l'alimentation de ladite unité. Cela permet non seulement l'utilisation de surfaces de membranes considérablement réduites par rapport aux autres procédés utilisant des unités de perméation pour ce type de séparation, mais également de récupérer le perméat en phase vapeur en aval de la membrane sous des pressions sensiblement plus élevées, ce qui réduit considérablement la puissance nécessaire à la compression de ce perméat.

Le procédé de la présente invention est donc particulièrement économe, à la fois en investissements et en consommation énergétique.

Enfin, le fait de traiter dans l'unité de perméation une phase soutirée latéralement sur la colonne à distiller, plutôt qu'une phase obtenue en tête de ladite colonne, permet d'avoir dans la phase ainsi traitée une teneur en méthanol supérieure et une teneur en eau inférieure à celles obtenues en tête de la colonne à distiller. Cela permet de récupérer un perméat plus riche en méthanol et moins riche en eau et par conséquent de meilleure qualité pour être recyclé vers la section d'éthérification.

Le procédé de séparation simultané du méthanol, de l'eau et du diméthyléther d'un mélange d'hydrocarbures tel qu'il a été décrit ci-dessous peut avantageusement être intégré dans un procédé de synthèse d'éthers à partir de méthanol et d'oléfines tertiaires présentes dans un mélange d'hydrocarbures pouvant avoir de 3 à 8 atomes de carbone (coupes C4 à C8 et/ou leurs mélanges).

Un tel procédé de synthèse d'éthers est décrit ci-dessous, en liaison avec la figure 2.

Le mélange d'hydrocarbures contenant les oléfines tertiaires nécessaires à la réaction est amené en phase liquide par la ligne 21. Il est mélangé au méthanol nécessaire à la réaction amené en phase liquide par la ligne 22 ainsi qu'au perméat condensé et refroidi de l'unité de pervaporation recyclé par la ligne 15. Ce perméat est pour l'essentiel constitué de l'excès de méthanol utilisé pour la réaction, ainsi que d'une fraction mineure, généralement moins de 0,5 %, des hydrocarbures n'ayant pas réagi présents dans l'effluent de la section réactionnelle.

Le mélange ainsi obtenu est introduit par la ligne 23 dans la section réactionnelle d'éthérification R, où les oléfines tertiaires et le méthanol sont convertis en éthers. La réaction secondaire d'éthérification du méthanol produit également dans cette section réactionnelle de faibles quantités d'eau et de diméthyléther qui doivent ensuite être éliminées au moins en partie.

L'effluent de la section réactionnelle est ensuite amené par la ligne 24 à une colonne à distiller D1. En fond de cette colonne, on récupère un effluent constitué pour l'essentiel de la totalité des éthers tertiaires produits (MTBE). Cet effluent peut également contenir certains des hydrocarbures les plus lourds présents dans l'effluent de la section réactionnelle. C'est notamment le cas lorsque les hydrocarbures constituant la charge de la section réactionnelle ont des nombres d'atomes de carbone différents (mélanges de coupes C4 à C8). Il faut noter que dans ce cas les hydrocarbures présents en fond de la colonne à distiller D1 peuvent généralement être utilisés comme constituants de l'essence sans avoir besoin d'être séparés des éthers produits. Enfin, selon la quantité de méthanol utilisée en excès, une fraction dudit méthanol peut également être présente dans l'effluent en fond de la colonne à distiller D1. La récupération de ce méthanol nécessitant un fractionnement supplémentaire, il est en général préférable de limiter l'excès de méthanol utilisé à des teneurs telles que tout l'excès de méthanol soit récupéré dans l'effluent de tête de la colonne à distiller D1. Cet effluent de tête comprend en outre la majeure partie des hydrocarbures n'ayant pas réagi. Il contient également la totalité de l'eau et du diméthyléther présents dans l'effluent de la section réactionnelle.

Une alternative consiste à remplacer la colonne à distiller D1 par une colonne de distillation catalytique D'1, ce qui permet d'augmenter les taux de conversion des oléfines tertiaires et/ou de réduire l'excès de méthanol employé. Les effluents de la colonne D'1 auront des compositions analogues à celles de ceux de la colonne D1 avec simplement des teneurs en méthanol généralement réduites.

L'effluent de tête de la colonne à distiller D1 (ou D'1) est ensuite envoyé par la ligne 1 vers le dispositif de mise en oeuvre du procédé de l'invention.

Il est donc admis dans la colonne à distiller D. Cette colonne produit en fond les hydrocarbures exempts de méthanol, de diméthyléther et d'eau.

La vapeur de tête de la colonne D est envoyée après condensation partielle vers le ballon B. Du ballon B on récupère par la ligne 4 une vapeur résiduelle contenant l'essentiel du diméthyléther et une fraction mineure du méthanol, de l'eau et des hydrocarbures les plus légers. Cette vapeur résiduelle peut par exemple être utilisée comme gaz combustible.

Du même ballon B, on récupère par la ligne 5 une phase liquide aqueuse constituée de l'essentiel de l'eau et d'une fraction mineure du méthanol avec des traces de diméthyléther et d'hydrocarbures dissous. Cette phase liquide aqueuse sera généralement brûlée ou envoyée vers une unité de traitement des eaux, vu son faible débit. Il est cependant possible de la fractionner par distillation de manière à obtenir d'une part de l'eau pouvant être rejetée et d'autre part du méthanol pouvant être recyclé vers la section réactionnelle.

Enfin du ballon B, on renvoie par la ligne 7 en reflux sur la colonne à distiller D une phase organique liquide constituée essentiellement d'hydrocarbures contenant de faibles quantités de méthanol, de diméthyléther et d'eau dissoutes.

Une fraction liquide est prélevée sur un soutirage latéral de la colonne à distiller D par la ligne 10 pour être envoyée à l'unité de pervaporation PV équipée d'une membrane sélectivement perméable au méthanol. Cette fraction liquide est prélevée préférentiellement au plateau de la colonne à distiller D où la concentration en méthanol est sensiblement maximale.

L'unité de pervaporation PV produit alors par la ligne 11 un rétentat appauvri en méthanol qui est recyclé vers un plateau inférieur à celui du soutirage latéral de la colonne à distiller D.

L'unité de pervaporation PV produit également un perméat recyclé au moins en partie par la ligne 15 vers la section réactionnelle R. Il peut aussi être recyclé au moins en partie dans la zone de distillation D'1 par les lignes 15 et 19, si cette zone est une zone de distillation catalytique. Comme mentionné précédemment ce perméat est pour l'essentiel constitué du méthanol utilisé en excès dans la section réactionnelle R. Il peut également, selon les conditions opératoires et les caractéristiques de la membrane de l'unité de pervaporation PV contenir des proportions variables d'eau, de diméthyléther et d'hydrocarbures. Les quantités de ces produits ainsi recyclées sont cependant suffisamment faibles pour ne pas avoir d'impact significatif sur le fonctionnement de la section réactionnelle R.

Les exemples qui suivent sont donnés à titre illustratif.

### EXEMPLE 1

Dans cet exemple une coupe C4 contenant du méthanol, de l'eau et du diméthyléther (voir composition tableaux 1-a et 1-b) est ramenée à de très faibles teneurs en composés oxygénés (<1ppm poids d'eau, <1ppm poids de méthanol, 10 ppm poids de diméthyléther) par le procédé de la présente invention d'une part (exemple 1-a) et par un procédé conventionnel constitué d'une étape de pervaporation suivie d'une étape de distillation (exemple 1-b) d'autre part.

### Exemple 1-a, procédé de l'invention (figure 1)

On utilise d'une part une colonne à distiller D en acier inoxydable de 100mm de diamètre et comportant 56 plateaux perforés espacés de 5cm et d'autre part un module de pervaporation en acier inoxydable equipé d'une membrane dont la couche sélective est constituée de poly(4)-vinyl pyridine réticulée par du 1,4 dibromobutane et qui est réalisée suivant la méthode décrite à l'exemple XVI du brevet US 5,152,898. Ce module est constitué d'un empilement de 2 éléments ayant chacun une surface utile de perméation de 0,1m². La surface totale de perméation est donc de 0,2 m².

Le mélange à séparer, ALIMENTATION, est introduit en phase liquide à une température de 78,0 °C dans la colonne à distiller D par la ligne 1 au plateau numéro 12. La colonne D est opérée sous une pression absolue de 22 bar, mesurée au ballon décanteur B. La température de la colonne D s'étale entre 115,5 °C en fond et 80,4 °C en tête.

La vapeur obtenue par la ligne 2 en tête de la colonne D est condensée partiellement puis décantée dans le ballon B. La température dans le ballon B est de 69,9°C. Du ballon B on soutire par la ligne 4 une phase gazeuse C3 et par la ligne 5 une phase liquide aqueuse EAU. La phase liquide organique obtenue par la ligne 6 est renvoyée en reflux en tête de la colonne D. Le taux de reflux (rapport du débit massique du reflux liquide en tête de la colonne D à celui de l' ALIMENTATION) est de 0,69.

En fond de la colonne D, on récupère en phase liquide l'essentiel de la coupe C4, purifiée, C4PURIFIES.

Au plateau numéro 8 de la colonne D on soutire une phase liquide, SOUTIRAGE, à une température de 100,2°C, que l'on refroidit jusqu'à la température de 50,0 °C et qui alimente ensuite par la ligne 10 l'unité de pervaporation PV. Cette unité PV produit par la ligne 13 un perméat en phase vapeur, PERMEAT, qui est recueilli après avoir été condensé, et un rétentat en phase liquide, RETENTAT, qui après réchauffage jusqu'à la température de 106,0 °C est renvoyé sur la colonne D au plateau numéro 18.

Le bilan matière (débits et compositions massiques des fluides ALIMENTATION, C3, EAU, C4PURIFIES, SOUTIRAGE, PERMEAT et RETENTAT) de ce procédé est indiqué dans le tableau 1-a.

**Tableau 1-a**

| Bilan matière du procédé de l'invention, exemple 1-a | | | | | | | |
|---|---|---|---|---|---|---|---|
| CORPS (%poids) | ALIMENTATION | C3 | EAU | C4-PURIFIES | SOUTIRAGE | PERMEAT | RETENTAT |
| C3 | 1,54 | 66,15 | 3,52 | 1,20 | 9,23 | 1,00 | 9,72 |
| C4 | 95,94 | 25,92 | 0,88 | 98,80 | 84,54 | 9,34 | 89,11 |
| Methanol | 2,44 | 2,64 | 48,72 | <1ppm | 5,74 | 89,27 | 0.68 |
| Eau | 0,05 | 0,53 | 45,16 | <1ppm | 0,02 | 0,34 | <1ppm |
| Diméthyléther | 0,03 | 4,76 | 1,72 | 10ppm | 0,47 | 0,05 | 0,49 |
| Débit (g.h⁻¹.m⁻²⁾ | 4000,0 | 21,3 | 3,3 | 3868,4 | 1872,3 | 107,0 | 1765,3 |

### Exemple 1-b, (comparatif)

Le procédé envisagé ici consiste en la simple succession d'une étape de pervaporation PV, destinée à extraire la majeure partie du méthanol du mélange à séparer, suivie d'une étape de distillation D, destinée à éliminer les composés oxygénés résiduels (traces de méthanol et de diméthyléther essentiellement, l'eau étant en quasi totalité extraite lors de l'étape de pervaporation).

On utilise la même colonne de distillation D que dans l'exemple 1-a, opérée sous une pression identique (22 bar au ballon décanteur B). Le module de pervaporation PV utilisé est équipé de la même membrane qu'à l'exemple 1-a mais il est cette fois constitué d'un empilement de 8 éléments ayant chacun une surface utile de perméation de 0,1 m². La surface totale de perméation est donc de 0,8 m². Dans ces conditions, on peut obtenir dans la coupe C₄ ainsi purifiée les mêmes teneurs en composés oxygénés que celles obtenues avec le procédé de l'invention dans l'exemple 1-a.

Le mélange à séparer, ALIMENTATION, identique à celui de l'exemple 1-a est admis en phase liquide dans l'unité de pervaporation PV à la température de 50,0 °C. L'unité PV produit un rétentat liquide, RETENTAT, alimentant la colonne de distillation D au plateau numéro 6 et un perméat en phase vapeur, PERMEAT, qui est recueilli après avoir été condensé.

La température de la colonne D s'étale entre 115,4 °C en fond et 97,5 °C en tête.

De manière analogue à l'exemple 1-a, la vapeur obtenue en tête de la colonne D est condensée partiellement puis décantée dans le ballon B. La température mesurée dans le ballon B est de 88,9 °C. Du ballon B on récupère une phase gazeuse, C3. La quasi-totalité de l'eau contenue dans l'ALIMENTATION ayant été extraite dans l'unité PV et se trouvant donc dans le PERMEAT, on ne receuille pas dans ce cas de phase liquide aqueuse au niveau du ballon B. On récupère par contre une phase liquide organique envoyée en reflux en tête de la colonne à distiller D. Le taux de reflux (rapport du débit massique du reflux liquide en tête de la colonne D à celui de l' ALIMENTATION) dans ce cas est de 0,57.

En fond de la colonne D on obtient la coupe C4 purifiée, C4PURIFIES.

Le bilan matière (débits et compositions massiques des fluides ALIMENTATION, C3, C4PURIFIES, PERMEAT et RETENTAT) de ce procédé est indiqué dans le tableau 1-b.

**Tableau 1-b**

| Bilan matière du procédé de l'exemple 1-b | | | | | |
|---|---|---|---|---|---|
| CORPS (%poids) | ALIMENTATION | C3 | C4-PURIFIES | PERMEAT | RETENTAT |
| C3 | 1,54 | 33,03 | 1,25 | 0,45 | 1,58 |
| C4 | 95,94 | 59,31 | 98,75 | 30,38 | 98,34 |
| Methanol | 2,44 | 4,87 | <1ppm | 67,74 | 0,05 |
| Eau | 0,05 | <1ppm | <1ppm | 1,42 | <1ppm |
| Diméthyléther | 0,03 | 2,79 | 10ppm | 0,01 | 0,03 |
| Débit (g.h⁻¹.m⁻²⁾ | 4000,0 | 40,0 | 3818,8 | 141,2 | 3858,8 |

La comparaison des résultats des exemples 1-a et 1-b illustre les avantages du procédé de l'invention. En effet pour un même mélange à traiter et une même pureté des hydrocarbures obtenus (mêmes teneurs en composés oxygénés résiduels), avec en outre le même type de membrane et une pression dans la zone de distillation ainsi qu'un nombre d'étages de distillation identiques, le procédé de l'invention offre:
- une surface de membrane considérablement réduite (0,2 m² au lieu de 0,8 m² ),
- une meilleure pureté du méthanol récupéré dans le PERMEAT (89,27 % poids au lieu de 67,74 % poids) avec notamment une teneur en eau moindre (0,34 % poids au lieu de 1,42 % poids) ce qui facilite son éventuelle utilisation dans un procédé d'éthérification.
- une moindre perte en hydrocarbures dans la vapeur C3 (21,3 g.h⁻¹ au lieu de 40,0 g.h⁻¹),
- en conséquence des deux points précédents, une quantité plus importante d'hydrocarbures purifiés récupérés (3868,4 g.h⁻¹ au lieu de 3818,8 g.h⁻¹).

### EXEMPLE 2

Cet exemple illustre le fonctionnement du procédé de l'invention lorsque le mélange à séparer n'est constitué que d'hydrocarbures et de méthanol, sans eau ni diméthyléther.

On utilise d'une part une colonne à distiller D en acier inoxydable de 100mm de diamètre et comportant 60 plateaux perforés espacés de 5cm et d'autre part un module de pervaporation en acier inoxydable equipé d'une membrane réalisée suivant la méthode décrite à l'exemple 2 du brevet allemand DE 4234521. Ce module est constitué d'un élément ayant une surface utile de perméation de 0,1m².

Un mélange d'hydrocarbures (coupe C4) contenant du méthanol, ALIMENTATION, est introduit en phase liquide à la température de 78.0°C, par la ligne 1 au plateau numéro 12 de la colonne de distillation D, représentée sur la figure 1. La colonne D est opérée sous une pression absolue de 22 bar, mesurée au ballon décanteur B. La température de la colonne D s'étale entre 115,1°C en fond et 73,7°C en tête.

Cette fois la vapeur obtenue par la ligne 2 en tête de la colonne D est entièrement condensée et renvoyée par les lignes 6 et 7 comme reflux liquide en tête de la colonne D. On ne prélève donc du ballon décanteur B ni phase vapeur par la ligne 4, ni phase liquide aqueuse par la ligne 5. La température du ballon décanteur B est de 64,1°C. Le taux de reflux (rapport du débit massique du reflux liquide en tête de la colonne D à celui de l' ALIMENTATION) est de 0,72.

En fond de la colonne D on récupère par la ligne 16 les hydrocarbures purifiés à moins de 1ppm poids de méthanol résiduel, C4PURIFIES.

Au plateau numéro 8 de la colonne D on soutire une phase liquide, SOUTIRAGE, à une température de 97,8°C, qui, après avoir été portée à la température de 50 °C, alimente par la ligne 10 l'unité de pervaporation PV. Cette unité PV produit par la ligne 13 un perméat en phase vapeur, PERMEAT, que l'on recueille après l'avoir condensé, et un rétentat en phase liquide, RETENTAT, qui, après avoir été porté à la température de 103,5°C, est renvoyé sur la colonne D, au niveau du plateau numéro 18.

Le bilan matière (débits et compositions massiques des fluides ALIMENTATION, C4PURIFIES, SOUTIRAGE, PERMEAT et RETENTAT) de ce procédé est indiqué dans le tableau 2.

Dans ce cas également l'emploi du procédé de l'invention s'avère avantageux. En effet la concentration en méthanol dans le SOUTIRAGE (5,74 % poids) est nettement supérieure à celle dans l'ALIMENTATION (2,44 % poids) mais également à celle mesurée dans le ballon décanteur B (3,68 % poids). L'alimentation de l'unité de pervaporation PV par le SOUTIRAGE latéral permet donc là encore de réduire considérablement la surface de membrane nécessaire.

**Tableau 2**

| Bilan matière du procédé de l'invention en l'absence d'eau et de diméthyléther | | | | | |
|---|---|---|---|---|---|
| CORPS (%poids) | ALIMENTATION | C4-PURIFIES | SOUTIRAGE | PERMEAT | RETENTAT |
| C3 | 1,54 | 1,54 | 12,73 | 0,18 | 13,42 |
| C4 | 96,02 | 98,46 | 81,53 | 1,12 | 85,91 |
| Methanol | 2,44 | <1ppm | 5,74 | 98,70 | 0,67 |
| Débit (g.h⁻¹.m⁻²⁾ | 4000,0 | 3901,0 | 1914,4 | 99,0 | 1815,4 |

### EXEMPLE 3

Cet exemple illustre le fonctionnement du procédé de l'invention lorsqu'on désire extraire les composés oxygénés d'une coupe C8 contenant du méthanol et du diméthyléther.

On utilise d'une part une colonne à distiller D en acier inoxydable de 100mm de diamètre et comportant 40 plateaux perforés espacés de 5cm et d'autre part un module de pervaporation en acier inoxydable equipé d'une membrane réalisée suivant la méthode décrite à l'exemple XVI du brevet US 5,152,898. La surface utile de perméation de ce module est de 0,0125 m².

Le mélange à séparer, ALIMENTATION, est introduit en phase liquide à une température de 120,0 °C dans la colonne à distiller D par la ligne 1 au plateau numéro 10. La colonne D est opérée sous une pression absolue de 3 bar, mesurée au ballon décanteur B. La température de la colonne D s'étale entre 182,2 °C en fond et 97,8°C en tête.

La vapeur obtenue par la ligne 2 en tête de la colonne D est condensée partiellement puis décantée dans le ballon B. La température dans le ballon B est de 64,8°C. Du ballon B on soutire par la ligne 4 une phase gazeuse DME. L'ALIMENTATION ne contenant pas d'eau, on ne recueille pas de phase liquide aqueuse. La phase liquide organique obtenue par la ligne 6 est renvoyée en reflux en tête de la colonne D. Le taux de reflux (rapport du débit massique du reflux liquide en tête de la colonne D à celui de l' ALIMENTATION) est de 0,56.

En fond de la colonne D on récupère en phase liquide l'essentiel de la coupe C8, purifiée, C8PURIFIES.

Au plateau numéro 2 de la colonne D on soutire une phase liquide, SOUTIRAGE, à une température de 99,1°C, que l'on refroidit jusqu'à la température de 50 °C et qui alimente ensuite par la ligne 10 l'unité de pervaporation PV. Cette unité PV produit par la ligne 13 un perméat en phase vapeur, PERMEAT, qui est recueilli après avoir été condensé, et un rétentat en phase liquide, RETENTAT, qui après réchauffage jusqu'à la température de 175,5 °C est renvoyé sur la colonne D au plateau numéro 14.

Le bilan matière (débits et compositions massiques des fluides ALIMENTATION, DME, C8PURIFIES, SOUTIRAGE, PERMEAT et RETENTAT) de ce procédé est indiqué dans le tableau 3.

**Tableau 3**

| Bilan matière du procédé de l'invention, exemple 3 | | | | | | |
|---|---|---|---|---|---|---|
| CORPS (%poids) | ALIMENTATION | DME | C8-PURIFIES | SOUTIRAGE | PERMEAT | RETENTAT |
| C7 | 1,00 | 4,32 | 1,02 | 6,62 | 0,07 | 14,74 |
| C8 | 96,55 | 7,42 | 98,98 | 30,53 | 0,35 | 68,02 |
| Methanol | 2,42 | 21,79 | <1ppm | 62,06 | 99,57 | 15,47 |
| Diméthyléther | 0,03 | 66,47 | <1ppm | 0,79 | 0,01 | 1,77 |
| Débit (g.h⁻¹.m⁻²⁾ | 4000,0 | 1,8 | 3901,3 | 174,9 | 96,9 | 78,0 |

## Revendications

1. Procédé de séparation de composés oxygénés comprenant en majeure partie du méthanol et éventuellement une mineure partie d'eau et/ou de diméthyléther d'un mélange d'hydrocarbures pouvant avoir de 3 à 8 atomes de carbone contenant lesdits composés oxygénés, comprenant une étape de distillation du mélange dans des conditions de pression et de température appropriées dans une zone de distillation, dans laquelle on recueille en tête de la zone de distillation un distillat gazeux comprenant du méthanol, le cas échéant de l'eau et/ou du diméthyléther, ainsi que des hydrocarbures qui est condensé au moins en partie et recyclé au moins en partie, on soutire latéralement de la zone de distillation une phase et on l'envoie dans des conditions adéquates dans une zone de perméation comprenant au moins une membrane sélectivement perméable au méthanol, on réalise une étape de perméation de ladite phase, on recueille en aval de la zone de perméation, au moins un perméat de préférence en phase vapeur enrichi en méthanol et en amont de la zone de perméation, au moins un rétentat, et on soutire en fond de la zone de distillation le mélange d'hydrocarbures purifiés.

2. Procédé selon la revendication 1 dans lequel on soutire latéralement la phase au niveau d'au moins un plateau de la zone de distillation dans lequel la concentration en méthanol est au moins égale à celle du méthanol dans le mélange d'hydrocarbures et de préférence dans lequel la concentration en méthanol est sensiblement maximale.

3. Procédé selon l'une des revendications 1 à 2 dans lequel la pression dans la zone de distillation est supérieure à 1 bar absolu , la température de tête est de 40 à 120°C et la température de fond est de 70 à 200°C et dans lequel la pression dans la zone de perméation est supérieure, égale ou inférieure à la pression dans la zone de distillation.

4. Procédé selon l'une des revendications 1 à 3 dans lequel le rétentat est recyclé dans des conditions adéquates dans la zone de distillation et avantageusement à un plateau inférieur à celui du soutirage latéral de la phase.

5. Procédé selon l'une des revendications 1 à 4 dans lequel le rétentat est recyclé dans des conditions adéquates au plateau dont la composition est sensiblement la même que celle du rétentat.

6. Procédé selon l'une des revendications 1 à 5 dans lequel on condense et on décante le distillat gazeux, on recueille une phase liquide organique éventuellement saturée en eau qui est recyclée en tête de la colonne avec un taux de reflux de 0,3 à 3 fois le débit massique du mélange d'hydrocarbures, éventuellement une phase aqueuse, et éventuellement une phase liquide ou gazeuse contenant du méthanol et du diméthyléther.

7. Procédé selon l'une des revendications 1 à 6 dans lequel la membrane est sensiblement perméable au diméthyléther et dans lequel on recueille le perméat contenant du méthanol et au moins en partie du diméthyléther.

8. Procédé selon l'une des revendications 1 à 7 dans lequel le mélange d'hydrocarbures purifiés en fond de distillation contient une teneur en eau inférieure à 10 ppm poids, de préférence inférieure à 1 ppm poids, une teneur en diméthyléther inférieure à 100 ppm, avantageusement inférieure à 50 ppm poids et une teneur en méthanol inférieure à 50 ppm poids et de préférence inférieure à 5 ppm poids.

9. Utilisation du procédé selon l'une des revendications 1 à 8 à un procédé d'éthérification d'une coupe hydrocarbonée contenant des hydrocarbures oléfiniques pouvant avoir de 3 à 8 atomes de carbone avec du méthanol, selon laquelle on fait réagir dans une zone réactionnelle ladite coupe avec le méthanol dans des conditions adéquates, on recueille un effluent contenant des éthers organiques tels que MTBE ou TAME, des hydrocarbures et des composés oxygénés comprenant du méthanol, éventuellement de l'eau et éventuellement du diméthyléther, on effectue une distillation dans des conditions adéquates permettant de recueillir lesdits éthers d'une part et ledit mélange d'hydrocarbures comprenant lesdits composés oxygénés d'autre part, on sépare ledit mélange par le procédé décrit selon l'une des revendications 1 à 8, et l'on recycle au moins en partie, le perméat obtenu enrichi en méthanol dans la zone réactionnelle.

10. Utilisation selon la revendication 9 dans laquelle la distillation est effectuée dans une zone de distillation catalytique et l'on recycle éventuellement au moins une partie du perméat dans ladite zone de distillation catalytique.

## Claims

1. A process for the separation of oxygenated compounds, containing mainly methanol and possibly a minor amount of water and/or dimethyl ether, from a mixture of hydrocarbons containing 3 to 8 carbon atoms containing the oxygenated compounds, comprising a distillation step of the mixture in a distillation step in a distillation zone under appropriate temperature and pressure conditions, wherein a gaseous distillate containing methanol, if the case arises water and/or dimethyl ether, and hydrocarbons, is taken overhead and at least partially condensed and at least partially recycled, a phase is extracted as a side stream from the distillation zone and sent under suitable conditions to a permeation zone comprising at least one membrane which is selectively permeable to methanol; a permeation step is carried out on said phase, at least one permeate is recovered downstream of the permeation zone, preferably as a vapour enriched in methanol and at least one residue is recovered upstream of the permeation zone, and the purified hydrocarbon mixture is extracted from the bottom of the distillation zone.

2. Process according to claim 1 wherein the phase is extracted as a side stream from at least one tray in the distillation zone where the methanol concentration is at least equal to that of the methanol in the hydrocarbon mixture, preferably to the tray where the methanol concentration is substantially maximal.

3. Process according to claim 1 or claim 2 wherein the pressure in the distillation zone is above 1 bar absolute, the head temperature is 40°C to 120°C and the bottom temperature is 70°C to 200°C, and wherein the pressure in the permeation zone is greater than, equal to or less than the pressure in the distillation zone.

4. Process according to any one of claims 1 to 3 wherein the residue is recycled to the distillation zone under suitable conditions, advantageously to a lower tray than that of the side stream extraction of said phase.

5. Process according to any one of claims 1 to 4 wherein the residue is recycled under suitable conditions to the tray where the composition is substantially the same as that of the residue.

6. Process according to any one of claims 1 to 5 wherein the gaseous distillate is decanted and condensed, an organic liquid phase which may be saturated with water is recovered and recycled to the head of the column with a reflux ratio of 0.3 to 3 times the mass flow rate of the hydrocarbon mixture, and an aqueous phase may be recovered and a liquid or gaseous phase containing methanol and dimethyl ether may be recovered.

7. Process according to any one of claims 1 to 6 wherein the membrane is substantially permeable to dimethyl ether and wherein the permeate containing methanol and at least a portion of the dimethyl ether is recovered.

8. Process according to any one of claims 1 to 7 wherein the purified hydrocarbon mixture from the bottom of the distillation zone has a water content of less than 10 ppm by weight, preferably less than 1 ppm by weight, a dimethyl ether concentration of less than 100 ppm, advantageously less than 50 ppm by weight, and a methanol concentration of less than 50 ppm by weight, preferably less than 5 ppm by weight.

9. Use of a process according to any one of claims 1 to 8 in a process for the etherification of a hydrocarbon cut comprising olefin hydrocarbons containing 3 to 8 carbon atoms with methanol, in which said cut is reacted with the methanol in a reaction zone under suitable conditions, an effluent is recovered which contains the organic ethers such as MTBE or TAME, hydrocarbons and oxygenated compounds comprising methanol, water if present and dimethyl ether if present, distillation is carried out under suitable conditions to recover said ethers and said hydrocarbon mixture including said oxygenated compounds, said mixture is separated using the process described in any one of claims 1 to 8, and at least a portion of the methanol-enriched permeate obtained is recycled to the reaction zone.

10. Use according to claim 9 wherein distillation is carried out in a catalytic distillation zone and at least a portion of the permeate is optionally recycled to said catalytic distillation zone.

## Patentansprüche

1. Verfahren zur Auftrennung sauerstoffhaltiger Verbindungen, die zum großen Teil Methanol, gegebenenfalls einen kleinen Teil Wasser und/oder Dimethyläther einer Mischung von Kohlenwasserstoffen, welche 3 bis 8 Kohlenstoffatome aufweisen können, welche diese sauerstoffhaltigen Verbindungen enthält, umfassen, daß eine Stufe der Destillation der Mischung unter geeigneten Druck- und Temperaturbedingungen in einer Destillationszone umfaßt, wobei man am Kopf der Destillationszone ein gasförmiges Destillat gewinnt, das Methanol, gegebenenfalls Wasser und/oder Dimethyläther sowie Kohlenwasserstoffe umfaßt, das wenigstens teilweise kondensiert und wenigstens teilweise rezykliert wird, man seitlich der Destillationszone eine Phase abzieht und sie unter geeigneten Bedingungen in eine Permeationszone einführt, welche wenigstens eine Membran umfaßt, die für Methanol selektiv permeabel ist, man eine Stufe der Permeation dieser Phase durchführt, man unterhalb der Permeationszone wenigstens ein Permeat vorzugsweise in gasförmiger, mit Methanol angereicherter Phase und oberhalb der Permeationszone wenigstens ein Retentat gewinnt und man am Boden der Destillationszone die gereinigte Kohlenwasserstoffmischung abzieht.

2. Verfahren nach Anspruch 1, wobei man seitlich die Phase bei wenigstens einem Boden der Destillationszone abzieht, bei dem die Konzentration an Methanol wenigstens gleich jener von Methanol in der Mischung von Kohlenwasserstoffen ist und vorzugsweise bei der die Konzentration an Methanol im wesentlichen maximal ist.

3. Verfahren nach einem der Ansprüche 1 bis 2, wobei der Druck in der Destillationszone über 1 bar absolut liegt, die Temperatur am Kopf 40 bis 120°C beträgt und die Temperatur am Boden 70 bis 200°C beträgt und wobei der Druck in der Permeationszone größer, gleich oder kleiner als der Druck in der Destillationszone ist.

4. Verfahren nach einem der Ansprüche 1 bis 3, wobei das Retentat unter geeigneten Bedingungen in die Destillationszone, in vorteilhafter Weise bei einem Boden unterhalb demjenigen des seitlichen Abziehens der Phase, zurückgeführt wird.

5. Verfahren nach einem der Ansprüche 1 bis 4, wobei das Retentat unter geeigneten Bedingungen zu dem Boden zurückgeführt wird, dessen Zusammensetzung im wesentlichen gleich der des Retentats ist.

6. Verfahren nach einem der Ansprüche 1 bis 5, wobei man das gasförmige Destillat kondensiert und dekantiert, man eine organische flüssige, gegebenenfalls mit Wasser gesättigte Phase gewinnt, die zum Kopf der Kolonne mit einem Rücklaufverhältnis von 0,3 bis 3 mal dem Massedurchsatz der Mischung von Kohlenwasserstoffen zurückgeführt wird, gegebenfalls eine wäßrige Phase und gegebenenfalls eine flüssige oder gasförmige Phase, welche Methanol und Dimethyläther enthält, gewinnt.

7. Verfahren nach einem der Ansprüche 1 bis 6, wobei die Membran für Dimethyläther deutlich permeabel ist und wobei man das Permeat, welches Methanol und wenigstens teilweise Dimethyläther enthält, gewinnt.

8. Verfahren nach einem der Ansprüche 1 bis 7, wobei die gereinigte Mischung von Kohlenwasserstoffen am Boden des Destillationsbodens einen Wassergehalt von unter 10 ppm, vorzugsweise von 1 ppm und einen Dimethyläthergehalt von unter 100 ppm, vorzugsweise von unter 50 ppm und einen Methanolgehalt von unter 50 ppm und vorzugsweise von unter 5 ppm, bezogen auf das Gewicht, enthält.

9. Verwendung des Verfahrens nach einem der Ansprüche 1 bis 8 bei einem Verfahren zur Verätherung einer kohlenwasserstoffhaltigen Charge mit Methanol, welche olefinische Kohlenwasserstoffe enthält, die 3 bis 8 Kohlenstoffatome aufweisen können, gemäß welchem man in einer Reaktionszone die Charge mit dem Methanol unter geeigneten Bedingungen umsetzt, man einen Abstrom gewinnt, welcher organische Äther, wie MTBE oder TAME, Kohlenwasserstoffe und sauerstoffhaltige Verbindungen, welche Methanol, gegebenenfalls Wasser und gegebenenfalls Dimethyhläther umfassen, enthält, man eine Destillation unter geeigneten Bedingungen durchführt, welche es erlauben, eines Teils die Äther und andererseits die Mischung von Kohlenwasserstoffen zu gewinnen, welche diese sauerstoffhaltigen Verbindungen enthält, man die Mischung durch das beschriebene Verfahren gemäß einem der Ansprüche 1 bis 8 auftrennt und man wenigstens teilweise das mit Methanol angereicherte, erhaltene Permeat in die Reaktionszone zurückführt.

10. Verwendung nach Anspruch 9, wobei die Destillation in einer katalytischen Destillationszone durchgeführt wird und man gegebenenfalls wenigstens einen Teil des Permeats in die katalytische Destillationszone zurückführt.
